## Europäisches Patentamt

(19) **European Patent Office**

**Office européen des brevets**

(11) Publication number: **0 122 986**
**B1**

(12) # EUROPEAN PATENT SPECIFICATION

(45) Date of publication of patent specification: **15.04.87**

(51) Int. Cl.⁴: **C 12 P 19/24**

(21) Application number: **83302298.1**

(22) Date of filing: **22.04.83**

(54) **Process for the treatment of glucose feedstock and for its enzymatic conversion to fructose.**

(43) Date of publication of application:
**31.10.84 Bulletin 84/44**

(45) Publication of the grant of the patent:
**15.04.87 Bulletin 87/16**

(84) Designated Contracting States:
**AT BE CH DE FR GB IT LI NL SE**

(56) References cited:
US-A-4 002 576
US-A-4 371 616

STARCH/STÄRKE, vol. 31, no. 12, 1979, pages 409-413, Verlag Chemie, GmbH, Weinheim DE; G. TEGGE: "Anreicherung und Gewinnung von Fructose aus Isosirup oder Invertzucker"

(73) Proprietor: **UOP Inc.**
**10 UOP Plaza Algonquin & Mt. Prospect Roads Des Plaines Illinois 60016 (US)**

(72) Inventor: **Rohrbach, Ronald Paul**
**21 W 755 Ravine Road**
**Forest Lake Illinois (US)**
Inventor: **Maliarik, Mary Jon**
**99, Little Melody Lane**
**Lake Forest Illinois (US)**

(74) Representative: **Hale, Stephen Geoffrey et al**
**J.Y. & G.W. Johnson Furnival House 14/18 High Holborn**
**London WC1V 6DE (GB)**

Courier Press, Leamington Spa, England.

# 0 122 986

**Description**

Fructose has been used as a sugar substitute in many applications. Increasing sugar prices spur increased fructose usage, and current fructose production is at an all-time peak.

The largest commercial source of fructose is glucose, an end product of starch hydrolysis, which is isomerized to fructose by enzymatic methods using glucose isomerase. Because the high enzyme cost dictates its reusability, the isomerization is not done homogeneously, where recovery of enzyme would be difficult and costly, but instead is performed heterogeneously, using glucose isomerase immobilized in some manner. That is, either cells containing glucose isomerase, or the enzyme itself, are physically and/or chemically bound to a support through which flows a glucose feedstock, with isomerization of glucose to fructose resulting from contact of the feedstock with the immobilized glucose isomerase system (IMGI).

It is highly desirable to have the IMGI as productive as possible. A measure of its productivity is its half-life, by which is meant the time necessary to reduce the activity of an IMGI to one-half its initial value. We have repeatedly observed that the half-life of an IMGI was substantially longer using a purified glucose feedstock than with a commercial feedstock. These observations led us to the discovery that the deleterious effect on IMGI half-life was directly associated with the presence in the feedstock of carbonyl-containing components which formed a precipitate with 2,4-dinitrophenylhydrazine. It was further discovered that removal of these components, or poisons, from the feedstock led to a substantial increase in the half-life, and thus the productivity, of IMGI. A crucial discovery leading to the invention described herein is that certain oxidizing agents very effectively alter these poisons, or deleterious components, chemically in a glucose feedstock, thereby rendering them innocuous and, in effect, removing the poisons initially present.

An object of this invention is therefore to increase the productivity of immobilized glucose isomerase systems.

It is disclosed in US—A—4,002,576 to regenerate a spent immobilized glucose isomerase composite comprising glucose isomerase adsorbed to porous particles of a magnesia-alumina carrier and having associated therewith carbonaceous materials and contaminants resulting from the use of the composite for the continuous isomerisation of glucose to fructose. A sodium hypochlorite solution is circulated through the spent particles, whilst they are fluidised in a fluidised bed, in order to remove substantially all the carbonaceous materials and contaminants. However, there is no disclosure of any special treatment of a glucose feedstock prior to its enzymatic conversion to fructose using such a composite.

According to the invention there is provided a process of treating a glucose feedstock prior to enzymatic conversion to fructose comprising contacting the feedstock with from 50 to 500 ppm of an oxidizing agent at a pH from 6 to 9 at a temperature from 10 to 80°C. In one specific embodiment the oxidizing agent is hydrogen peroxide. In one or more specific and preferred embodiment the oxidizing agent is hydrogen peroxide and is used at a pH from 7.5 to 9. It has been found that such treatment substantially increases the productivity of the enzyme glucose isomerase when used in an immobilized enzyme system, such increasing productivity being manifested by an increased half-life of the IMGI.

The invention therefore includes a process for the production of fructose by enzymatic conversion of a glucose feedstock using glucose isomerase, especially in the form of IMGI, in which the feedstock is pretreated with an oxidizing agent at a pH of from 6 to 9 and a temperature of from 10 to 80°C.

The problem which this invention solves is that commercial glucose feedstocks for fructose formation via IMGI frequently contain poisons which substantially reduce the productivity of IMGI as manifested by its half-life. For example, if an IMGI has a half-life of 70 to 80 days with a purified glucose feedstock, the IMGI may have a half-life of only from about 25 to 40 days with a commercial glucose feedstock. For the purpose of this application, the term "poisons" refers to materials found in commercial glucose feedstocks which contribute to a decrease in half-life of immobilized glucose isomerase.

A characteristic common to all glucose feedstocks exhibiting a deleterious effect on the half-life is the presence of components in minor amounts which form precipitates with 2,4-dinitrophenylhydrazine. Although the nature of these minor components is not known with certainty, it was possible qualitatively to correlate their amount, via their derivatives with 2,4-dinitrophenylhydrazine, with the extent of the reduction in half-life of IMGI. It was found from that correlation that the greater the amount in the feedstock of components which form derivatives with 2,4-dinitrophenylhydrazine, the less the half-life of the IMGI was. It also was observed that when feedstocks in which such components were initially absent were treated so as to induce formation of these components, the resulting feedstocks substantially reduced the half-life of IMGI.

An inference which may be drawn from these observations is that the minor components forming precipitates with 2,4-dinitrophenylhydrazine are themselves poisons, or that the presence of the poisons is associated with the presence of the minor components. Although it is unknown which inference is the more correct one, this invention may be successfully practiced nonetheless. Similarly, although at least one of the minor components may be a 3-deoxyhexosone, it is not known unequivocally that this is present when precipitates form with 2,4-dinitrophenylhydrazine, nor is its effect as a poison independently known.

It was subsequently discovered that treating the feedstock containing the poisons with certain oxidizing agents destroys the aforementioned minor components, with the treated feedstock showing no deleterious effect on enzyme productivity. This discovery led to the process of this invention, which is

2

essentially a method of removing certain minor components from a glucose feedstock, components whose presence have a deleterious effect on the productivity of the IMGI.

In the process of this invention glucose feedstocks are treated with an effective amount of an oxidizing agent. Among the oxidizing agents which may be used in the process of this invention are hydrogen peroxide, other peroxides, hypohalites, especially hypochlorites and hypobromites, perhalates, especially perchlorates and perbromates, and persulfates. Hydrogen peroxide is a highly preferred oxidizing agent. Other preferred oxidizing agents include the alkali metal and alkaline earth metal salts of hypochlorites and perchlorates.

The amount of oxidizing agent effective in the practice of this invention depends somewhat on the oxidizing agent, the amount of poison present in the feed, and the pH at which treatment is performed. In particular, when hydrogen peroxide is used at a pH from 7.5 to 9, an amount from 50 to 250 ppm constitutes an effective amount, and an amount from 100 to 200 ppm is even more desirable. As the pH decreases, the amount necessary to be effective in the practice of this invention increases.

As previously stated, the process of this invention is pH dependent. It is found that the lower the pH, the greater must be the amount of oxidizing agent used. On the other hand, it is found that at a pH greater than 9 there are formed color bodies which are undesirable from a commercial aspect. Thus, a pH range from 6 to 9 is used in the practice of this invention, and the range from 7.5 to 9 is preferred.

The contact time is dependent upon the temperature, pH, the amount of oxidizing agent used, and the amount of undesirable component present. A temperature from 10 to 80°C may be employed, but a range from 50 to 70°C is preferred in order to minimize contact time without adversely affecting the feedstock. At a pH of about 9 with about 200 ppm hydrogen peroxide, a contact time of about 90 minutes at 20°C, or 20 minutes at 60°C, is adequate to maximize the beneficial effects of the process of this invention. It is to be understood that at a different pH and/or using a different oxidizing agent, the necessary contact time may differ from that stated above, but can be readily determined through simple experimentation.

Where it is necessary to remove residual oxidizing agent prior to contacting the glucose feedstock with IMGI, this may be done by treating the feedstock with bisulfite, sulfite, charcoal, iron (ferrous or ferric), or catalase. For example, feedstock with residual oxidizing agent may be passed through a bed of charcoal. As another example, residual oxidizing agent may be removed by adding a sulfite to the glucose feedstock. When this latter mode of treatment is chosen, it is found that from 100 to 1000 ppm of sulfite is preferred to be added.

The examples below are merely illustrative of this invention and the discovery underlying it.

In all the examples the IMGI used was a preparation in which glucose isomerase was immobilized onto a support matrix composed of alumina impregnated with polyethylenimine subsequently cross-linked with excess glutaraldehyde so as to furnish excess pendant aldehyde groups. The reactors used were of the fixed bed type operated at 60°C with feedstock in an upflow mode and a space velocity sufficient to afford 42% fructose in the effluent.

Example I

In this example two reactors, each containing IMGI, were run in series. The feedstock was 45% weight/weight purified glucose (Cerelose—Registered Trade Mark) containing 0.1% sodium sulfite and 7 ppm sodium omadine at pH 8.0. The feedstock gave no precipitate when treated with 2,4-dinitrophenylhydrazine. It had been found by independent experimentation that passage of the purified feedstock through the IMGI caused formation of undesirable minor components. In this example the normal mode of operation consisted of passing the feedstock through the first reactor, then using the effluent from the first reactor as the feed for the second reactor under otherwise identical operating conditions. In this mode the IMGI in the second reactor performed only a small chemical change to 48% fructose, since the feed to it, at 42% fructose, was already near equilibrium for the liquid hourly space velocity used. However, the IMGI in the second reactor was exposed to poisons formed in the first reactor. Periodically, the second reactor was fed with the purified glucose feedstock and its activity measured. This was done solely in order to determine the half-life of its IMGI. It was found that the half-life of the IMGI in the first reactor was about 144 days, whereas the half-life of the IMGI in the second reactor was about 84 days.

This demonstrates the effect of poisons in the feedstock on the half-life of IMGI.

Example II

Commercial feedstock containing operationally appreciable amounts of poisons, as determined by formation of 2,4-dinitrophenylhydrazine derivatives, was treated with varying amounts of hydrogen peroxide at pH 9 for 90 minutes at room temperature. When the peroxide was used at a concentration greater than about 100 ppm no observable precipitate formed. When the peroxide was used at levels between 25 and 50 ppm an observable precipitate formed. When peroxide was absent a fairly heavy precipitate formed. Thus, poisons were present at a level requiring at least 50 to about 100 ppm of oxidizing agent in this particular feedstock.

Example III

A portion of a commercial feedstock was treated for 90 minutes at ambient temperature and pH 9.0 with 200 ppm hydrogen peroxide. Excess peroxide was destroyed by addition of 1000 ppm sodium sulfite and the pH was adjusted to 8.2. Another portion of the feedstock was treated identically except hydrogen peroxide was omitted. The two feedstocks were used in two reactors operating in tandem to determine half-life of the IMGI. Results are given below.

|  | Hydrogen peroxide treated feed | Untreated feed |
|---|---|---|
| Initial activity (units per gram) | 1500 | 1370 |
| Half-life (days) | 74 | 38 |

Thus, hydrogen peroxide treatment increases the half-life of IMGI by a factor of almost two relative to untreated material.

## Claims

1. A process of treating a glucose feedstock prior to enzymatic conversion to fructose, characterised in that the feedstock is contacted with from 50 to 500 ppm of an oxidizing agent at a pH from 6 to 9 at a temperature from 10 to 80°C.

2. A process as claimed in claim 1, characterised in that the oxidizing agent is selected from peroxides, hypohalites, perhalates, and persulfates.

3. A process as claimed in claim 2, characterised in that the oxidizing agent is hydrogen peroxide.

4. A process as claimed in claim 2, characterised in that the oxidizing agent is a hypochlorite.

5. A process as claimed in claim 2, characterised in that the oxidizing agent is a perchlorate.

6. A process as claimed in any of claims 1 to 5, characterised in that the pH is from 7.5 to 9.

7. A process as claimed in any of claims 1 to 6, characterised in that the temperature is from 50 to 70°C.

8. A process as claimed in any of claims 1 to 7, characterised in that the amount of oxidizing agent is from 50 to 250 ppm.

9. A process for the production of fructose by enzymatic conversion of a glucose feedstock using glucose isomerase, characterised in that the glucose feedstock is pretreated by a process as claimed in any of claims 1 to 8.

10. A process as claimed in claim 9, characterised in that residual oxidizing agent is removed prior to enzymatic conversion of the glucose feedstock to fructose.

## Patentansprüche

1. Verfahren zur Behandlung eines Glucoseeinsatzmaterials vor der enzymatischen Umwandlung zu Fructose, dadurch gekennzeichnet, dass man das Einsatzmaterial bei pH 6 bis 9 und einer Temperatur von 10 bis 80°C mit 50 bis 500 ppm eines Oxydationsmittels in Berührung bringt.

2. Verfahren nach Anspruch 1, dadurch gekennzeichnet, dass man das Oxydationsmittel unter Peroxyden, Hypohalogeniten, Perhalogenaten und Persulfaten auswählt.

3. Verfahren nach Anspruch 2, dadurch gekennzeichnet, dass das Oxydationsmittel Wasserstoffperoxyd ist.

4. Verfahren nach Anspruch 2, dadurch gekennzeichnet, dass das Oxydationsmittel ein Hypochlorit ist.

5. Verfahren nach Anspruch 2, dadurch gekennzeichnet, dass das Oxydationsmittel ein Perchlorat ist.

6. Verfahren nach einem der Ansprüche 1 bis 5, dadurch gekennzeichnet, dass der pH 7,5 bis 9 beträgt.

7. Verfahren nach einem der Ansprüche 1 bis 6, dadurch gekennzeichnet, dass die Temperatur bei 50 bis 70°C liegt.

8. Verfahren nach einem der Ansprüche 1 bis 7, dadurch gekennzeichnet, dass die Menge Oxydationsmittel 50 bis 250 ppm beträgt.

9. Verfahren zur Herstellung von Fructose durch enzymatische Umwandlung eines Glucoseeinsatzmaterials mittels Glucoseisomerase, dadurch gekennzeichnet, dass man das Glucoseeinsatzmaterial gemäss einem Verfahren nach einem der Ansprüche 1 bis 8 vorbehandelt.

10. Verfahren nach Anspruch 9, dadurch gekennzeichnet, dass man restliches Oxydationsmittel vor der enzymatischen Umwandlung des Glucoseeinsatzmaterials zu Fructose entfernt.

## Revendications

1. Un procédé pour le traitement d'une charge d'alimentation de glucose avant son isomérisation enzymatique en fructose, caractérisé en ce que l'on met en contact la charge d'alimentation avec de 50 à 500 ppm d'un oxydant à un pH compris entre 6 et 9 et à une température comprise entre 10 et 80°C.

2. Un procédé selon la revendication 1, caractérisé en ce que l'oxydant est choisi parmi les peroxydes, les hypohalogénites, les perhalogénates et les persulfates.

3. Un procédé selon la revendication 2, caractérisé en ce que l'oxydant est l'eau oxygénée.

4. Un procédé selon la revendication 2, caractérisé en ce que l'oxydant est un hypochlorite.

5. Un procédé selon la revendication 2, caractérisé en ce que l'oxydant est un perchlorate.

6. Un procédé selon l'une quelconque des revendications 1 à 5, caractérisé en ce que le pH est compris entre 7,5 et 9.

7. Un procédé selon l'une quelconque des revendications 1 à 6, caractérisé en ce que la température est comprise entre 50 et 70°C.

8. Un procédé selon l'une quelconque des revendications 1 à 7, caractérisé en ce que la quantité d'oxydant est comprise entre 50 et 250 ppm.

9. Un procédé pour la production de fructose par isomérisation enzymatique d'une charge d'alimentation de glucose utilisant la glucose isomérase, caractérisé en ce que la charge d'alimentation de glucose est prétraitée par un procédé selon l'une quelconque des revendications 1 à 8.

10. Un procédé selon la revendication 9, caractérisé en ce que l'oxydant résiduel est éliminé avant l'isomérisation enzymatique de la charge d'alimentation de glucose en fructose.